# EUROPEAN PATENT SPECIFICATION

(11) **EP 0 475 370 B1**
(45) Date of publication and mention of the grant of the patent: **06.03.1996**
(21) Application number: 91115315.3
(22) Date of filing: 10.09.1991
(51) Int. Cl.: H04N 5/225, A61B 1/04, H01L 31/0203

(54) **Compact imaging apparatus for electronic endoscope with improved optical characteristics**
Kompaktes Bilderzeugungsgerät mit verbesserten optischen Eigenschaften für elektronische Endoskope
Appareil compact de formation d'images avec des caractéristiques optiques améliorées pour des endoscopes électroniques

(30) Priority: 10.09.1990 JP 237121/90
(43) Date of publication of application: 18.03.1992
(73) Proprietor: KABUSHIKI KAISHA TOSHIBA, Kawasaki-shi, Kanagawa-ken 210, Tokyo (JP)
(72) Inventor: Kameishi, Wataru, Ootawara-shi, Tochigi-ken (JP)
(74) Representative: Blumbach, Kramer & Partner

(56) References cited:
- EP-A- 0 325 525
- DE-A- 3 715 417
- GB-A- 2 165 092
- US-A- 3 757 127
- US-A- 5 021 888
- US-A- 5 040 069
- PATENT ABSTRACTS OF JAPAN vol. 7, no. 239 (E-206)(1384) 25 October 1983 & JP-A-58 128 762
- PATENT ABSTRACTS OF JAPAN vol. 9, no. 10 (E-290)(1733) 17 January 1985 & JP-A-59 161 078

## Description

### BACKGROUND OF THE INVENTION

### Field of the Invention

The present invention relates to an imaging apparatus to be mounted on a scope end section of an electronic endoscope.

### Description of the Background Art

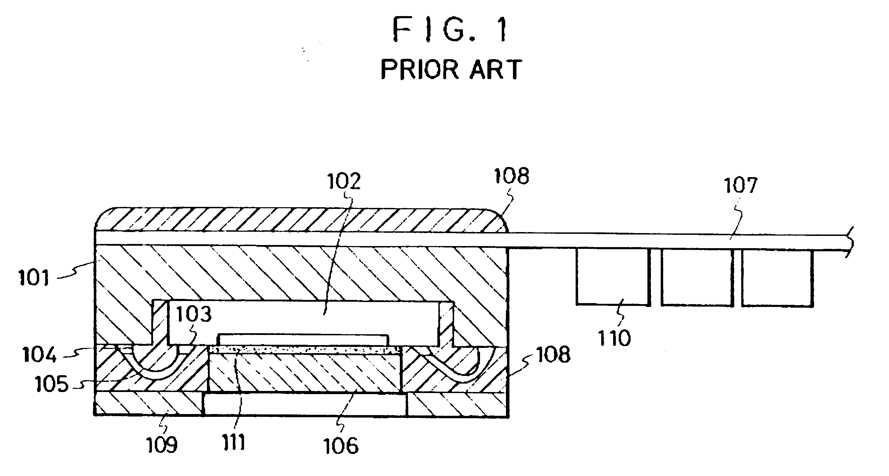

Conventionally, an imaging apparatus for an electronic endoscope has a configuration shown in Fig. 1. In this conventional imaging apparatus of Fig. 1, a CCD (charge coupled device) 102 is fixed on one side of a chip carrier 101 made from a ceramic substrate, where pads 103 provided on the CCD 102 and pads 104 provided on the chip carrier 101 are connected by bonding wires 105. Then, a cover glass 106 containing an optical filter is attached on an imaging surface of the CCD 102 by means of an adhesive resin 111 placed therebetween, while the other side of the chip carrier 101 is attached to a flexible print substrate 107 such that they are electrically connected. In addition, the above described structure formed by the chip carrier 101, the CCD 102, the bonding wires 105, the coyer glass 106, and the flexible print substrate 107 are covered by a mold resin 108, while the bonding wires 105 are protected by metallic frames 109 attached on the mold resin 108 over the locations of the bonding wires 105. Moreover, on a part of the flexible print substrate 107 not covered by the mold resin 108, chip components 110 are provided. With this configuration, the imaging apparatus is mounted on a scope end section of an electronic endoscope.

However, such a conventional imaging apparatus for an electronic endoscope has been associated with the following problems.

First, a conventional imaging apparatus with a configuration shown in Fig. 1 involves a number of piled up layers such as those for a height of the bonding wires 105, a distance between the bonding wires 105 and the metallic frames 109, a thickness of the chip carrier 101, a thickness of the metallic frames 109, a thickness of the flexible print substrate 107, and a thickness of the mold resin 108, so that a further thinning of a thickness of such an imaging apparatus has been practically impossible, and this in turn obstructed the further thinning of the scope end section of the electronic endoscope.

Secondly, the cover glass 106 is attached on the imaging surface of the CCD 102 by means of the adhesive resin 111 placed therebetween, such that micro-lenses (not shown) provided on the imaging surface of the CCD 102 are buried inside the adhesive resin 111. Here, because the index of refraction of the adhesive resin 111 is very close to that of the micro-lenses the light beam will hardly be refracted at a boundary between the adhesive resin 111 and the micro-lenses. As a consequence, an effect of improving a sensitivity of the CCD 102 due to the presence of the micro-lenses cannot be obtained.

Thirdly, the cover glass 106 cannot be made larger because of the possible interference between the cover glass 106 and the pads 103 of the CCD 102. As a result, it has been impossible to keep the reflections by the side surfaces of the cover glass 106 out of the imaging surface of the CCD 102.

Finally, in the imaging apparatus for an electronic endoscope, it is necessary to arrange the imaging surface of the CCD 102 to be perpendicular with respect to a light beam axis of an incident light beam. However, in the conventional imaging apparatus for an electronic endoscope, the cover glass 106 is attached on the imaging surface of the CCD 102 by means of the adhesive resin 111 placed therebetween as already mentioned above, so that it is difficult for the imaging surface of the CCD 102 to be arranged accurately in a desired direction because it is difficult to accurately control the thickness cf the adhesive resin 111 during its hardening process.

EP-A-0 325 525 discloses an imager for an endoscope, in which thickness is reduced by using an imaging element which is so thin that it is transparent. This element faces away from the incident light.

JP-A-1 161 775 discloses a stepped substrate structure. The glass cover covers the whole substrate and not just the stepped window portion.

### SUMMARY OF THE INVENTION

It is therefore an object of the present invention to provide an imaging apparatus for an electronic endoscope capable of realizing a compact size and improved optical characteristics.

According to one aspect of the present invention there is provided an imaging apparatus for an electronic endoscope as claimed in claim 1.

According to another aspect of the present invention there is provided an imaging apparatus for an electronic endoscope as claimed in claim 7.

Other features and advantages of the present invention will beccme apparent from the following description taken in conjunction with the accompanying drawings.

### BRIEF DESCRIPTION OF THE DRAWINGS

Fig. 1 is a cross sectional view of a conventional imaging apparatus for an electronic endoscope.

Figs. 2(A) and 2(B) are a plan view and a cross sectional view of a first embodiment of an imaging apparatus for an electronic endoscope according to the present invention.

Figs. 3(A) and 3(B) are a plan view and a cross sectional view of a second embodiment of an imaging apparatus for an electronic endoscope according to the present invention.

Fig. 4 is a cross sectional view of a third embodiment of an imaging apparatus for an electronic endoscope according to the present invention.

### DETAILED DESCRIPTION OF THE PREFERRED EMBODIMENTS

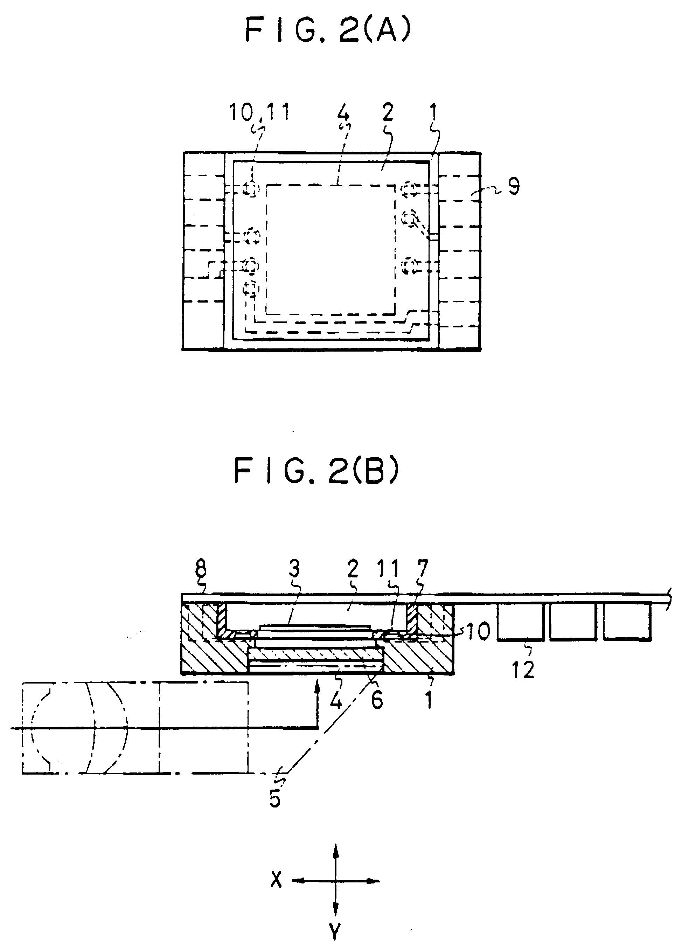

Referring now to Figs. 2(A) and 2(B), a first embodiment of an imaging apparatus for an electronic endoscope according to the present invention will be described in detail.

This first embodiment is a case in which an imaging surface 3 of an imaging element such as a CCD (charge coupled device) 2 is provided in parallel to a scope axis direction X for a scope end section.

In this first embodiment, the imaging apparatus is formed by a ceramic substrate 1 having a central plate portion with a window 4 and side bank portions projecting upwards, where the CCD 2 is mounted on an upper side of the central plate portion of the ceramic substrate 1 by a face-down bonding such that the imaging surface 3 of the CCD 2 is facing toward the window 4 of the central plate portion of the ceramic substrate 1.

In the window 4 of the central plate portion of the ceramic substrate 1, there is provided a cover glass 6 such that a light beam entering from an object optical system 5 in a scope diameter direction Y toward the window 4 passes through this cover glass 6 so as to be transmitted to the imaging surface 3 of the CCD 2.

The CCD 2 is supported on the ceramic substrate 1 by means of a mold resin 7 inserted therebetween except for the location of the imaging surface 3, such that an air layer is formed between the cover glass 6 and the imaging surface 3 of the CCD 2.

Moreover, the ceramic substrate 1 has multiple layers of wiring embedded and the embedded wirings are lead to electrodes 9 formed on the side bank portions of the ceramic substrate 1, such that the wiring in the ceramic substrate 1 can be electrically connected with a flexible print substrate 8 attached on the side bank portions of the ceramic substrate 1 through the electrodes 9. This flexible print substrate 8 also has a number of chip components 12 attached besides the ceramic substrate 1.

Furthermore, the central plate portion of the ceramic substrate 1 has pads 10 connected with the wirings inside the ceramic substrate 1 on the upper side of the central plate portion, while the CCD 2 has pads 11 in a vicinity of the imaging surface 3 such that when the CCD 2 is mounted on the central plate portion of the ceramic substrate 1 by the face-down bonding, the pads 10 of the ceramic substrate 1 and the pads 11 of the CCD 2 are electrically connected by a mutual compression bonding. Here, the pads 10 of the ceramic substrate 1 and the pads 11 of the CCD 2 may be formed with metallic or solder bumps provided such that the mutual compression bonding between the pads 10 of the ceramic substrate 1 and the pads 11 of the CCD 2 can be realized by a thermocompression bonding of the bumps.

Thus, according to this first embodiment, the electrical connection between the pads 10 of the ceramic substrate 1 and the pads 11 of the CCD 2 is obtained by mounting the CCD 2 on the central plate portion of the ceramic substrate 1 by face-down bonding, so that no bonding wire for providing this electrical connection is necessary.

Consequently, this imaging apparatus of the first embodiment can be made thinner by a height of the bonding wires compared with a conventional imaging apparatus involving the bonding wires, which in turn contributes to the improvement of the maneuverability of the electronic endoscope in which the imaging apparatus of this first embodiment is used.

Moreover, in this first embodiment, there is no need to provide a covering of the bonding wires by the mold resin as well as a protection of the bonding wires by metallic frames, unlike a conventional imaging apparatus involving the bonding wires for which both of these are required. As a consequence, a configuration of the imaging apparatus can be made simpler than that of a conventional imaging apparatus as a number of components involved can be reduced.

Furthermore, because of the absence of the bonding wires, the cover glass 6 can be made sufficiently larger than the imaging surface 3 of the CCD 2, such that the reflections by the side surfaces of the cover glass 6 can be kept out of the imaging surface 3 of the CCD 2.

Also, the S/N ratio can be improved in this imaging apparatus as a result of the micro-lense effect obtained by the difference between the index of refraction of micro-lenses (not shown) provided on the imaging surface 3 of the CCD 2 and that of the air layer provided between the cover glass 6 and the imaging surface 3 of the CCD 2.

In addition, the application of the ultrasonic waves conventionally used in making the wire bonding of the pads becomes unnecessary, such that the wirings inside the ceramic substrate 1 can be designed freely without considering the effect due to the application of the ultrasonic waves.

Also, because of the use of the face-down bonding between the CCD 2 and the ceramic substrate 1 in which the imaging surface 3 of the CCD 2 can be arranged highly accurately, there is no adhesive layer involved between the CCD 2 and the ceramic substrate 1, such that the accuracy for arranging the imaging surface 3 of the CCD 2 to be perpendicular with respect to an incident light beam axis of the object optical system 5 can be improved, and therefore the optical characteristics of the imaging apparatus can be improved.

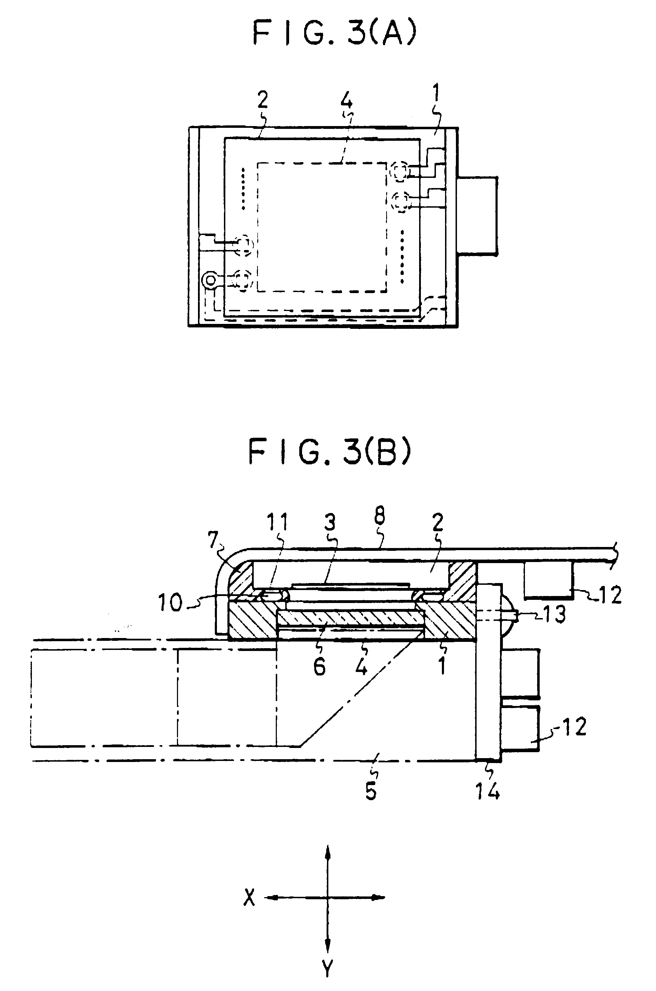

Referring now to Figs. 3(A) and 3(B), a second embodiment of an imaging apparatus for an electronic endoscope according to the present invention will be described in detail. Here, those elements which are equivalent to corresponding elements in the first embodiment described above are given the same reference numerals in the figures.

This second embodiment is also a case in which an imaging surface 3 of a CCD 2 is provided in parallel to a scope axis direction X for a scope end section.

In this second embodiment, the imaging apparatus is formed by a ceramic substrate 1 having a central plate portion with a window 4, where the CCD 2 is mounted on an upper side of the central plate portion of the ceramic substrate 1 by a face-down bonding such that the imaging surface 3 of the CCD 2 is facing toward the window 4 of the central plate portion of the ceramic substrate 1.

In the window 4 of the central plate portion of the ceramic substrate 1, there is provided a cover glass 6 such that a light beam entering from an object optical system 5 in a scope diameter direction Y toward the window 4 passes through this cover glass 6 so as to be transmitted to the imaging surface 3 of the CCD 2.

The CCD 2 is supported on the ceramic substrate 1 by means of a mold resin 7 inserted therebetween except for the location of the imaging surface 3, such that an air layer is formed between the cover glass 6 and the imaging surface 3 of the CCD 2.

Moreover, the ceramic substrate 1 has multiple layers of wiring embedded and the embedded wirings are lead to a junction board 13 attached on a side face of the ceramic substrate 1, where the junction board 13 is pierced into a print substrate 14 such that the wiring in the ceramic substrate 1 can be electrically connected with the print substrate 14 having a number of chip components 12 attached beside the ceramic substrate 1.

In addition, a flexible print substrate 8 which also has a number of chip components 12 attached is provided over the upper side of the CCD 2 and the side face of the ceramic substrate 1.

Furthermore, the central plate portion of the ceramic substrate 1 has pads 10 connected with the wirings inside the ceramic substrate 1 on the upper side of the central plate portion, while the CCD 2 has pads 11 in a vicinity of the imaging surface 3 such that when the CCD 2 is mounted on the central plate portion of the ceramic substrate 1 by the face-down bonding, the pads 10 of the ceramic substrate 1 and the pads 11 of the CCD 2 are electrically connected by a mutual compression bonding.

Thus, according to this second embodiment, the imaging apparatus can be further shortened in the scope axis direction X compared with the first embodiment described above, while maintaining the other advantages similar to those described above for the first embodiment.

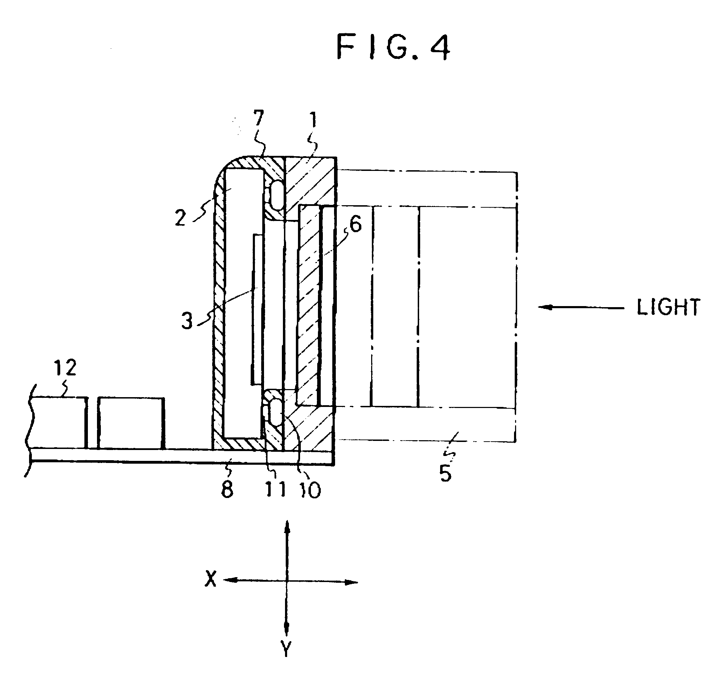

Referring now to Fig. 4, a third embodiment of an imaging apparatus for an electronic endoscope according to the present invention will be described in detail. Here, those elements which are equivalent to corresponding elements in the first embodiment described above are given the same reference numerals in the figures.

This third embodiment is a case in which an imaging surface 3 of a CCD 2 is provided along a scope diameter direction Y perpendicular to a scope axis direction X for a scope end section.

In this third embodiment, the imaging apparatus is formed by a ceramic substrate 1 having a central plate portion with a window 4, where the CCD 2 is mounted on an upper side of the central plate portion of the ceramic substrate 1 by a face-down bonding such that the imaging surface 3 of the CCD 2 is facing toward the window 4 of the central plate portion of the ceramic substrate 1.

In the window 4 of the central plate portion of the ceramic substrate 1, there is provided a cover glass 6 such that a light beam entering from an object optical system 5 in the scope axis direction X toward the window 4 passes through this cover glass 6 so as to be transmitted to the imaging surface 3 of the CCD 2.

The CCD 2 is supported on the ceramic substrate 1 by means of a mold resin 7 inserted therebetween except for the location of the imaging surface 3, such that an air layer is formed between the cover glass 6 and the imaging surface 3 of the CCD 2.

Moreover, the ceramic substrate 1 has multiple layers of wiring embedded and the embedded wirings are lead to a side face of the ceramic substrate 1, such that the wiring in the ceramic substrate 1 can be electrically connected with a flexible print substrate 8 attached on the side face of the ceramic substrate 1. This flexible print substrate 8 also has a number of chip components 12 attached beside the ceramic substrate 1.

Furthermore, the central plate portion of the ceramic substrate 1 has pads 10 connected with the wirings inside the ceramic substrate 1 on the upper side of the central plate portion, while the CCD 2 has pads 11 in a vicinity of the imaging surface 3 such that when the CCD 2 is mounted on the central plate portion of the ceramic substrate 1 by the face-down bonding, the pads 10 of the ceramic substrate 1 and the pads 11 of the CCD 2 are electrically connected by a mutual compression bonding.

Thus, according to this third embodiment, in addition to the other advantages similar to those described above for the first embodiment, the imaging apparatus can be further shortened in the scope axis direction X compared with the first embodiment described above, so that it becomes possible to shorten a hard portion in a scope end section of an electronic endoscope in which the imaging apparatus of this third embodiment is used, which in turn contributes to the improved maneuverability of the electronic endoscope.

It is to be noted that, besides those already mentioned above, many modifications and variations of the above embodiments may be made without departing from the novel and advantageous features of the present invention. Accordingly, all such modifications and variations are intended to be included within the scope of the appended claims.

## Claims

1. An imaging apparatus for an electronic endoscope, comprising:
a rigid circuit substrate (1), having substrate connection pads (10), electrodes (9) connected to the connection pads, and a window (4) through which a light beam passes;
an imaging element (2) having an imaging surface (3) for receiving the light beam through the window (4) of the rigid circuit substrate (1), and element connection pads (11), both the imaging surface and element connection pads being located on one side of the imaging element (2), the imaging element (2) being attached on the rigid circuit substrate (1) such that the substrate connection pads (10) of the rigid circuit substrate (1) and the element connection pads (11) of the imaging element (2) are electrically connected by a mutual compression bonding between the rigid circuit substrate (1) and the imaging element (2);
CHARACTERIZED IN THAT
an insulative flexible print substrate (8) is connected with the electrodes (9) of the rigid circuit substrate (1);
the window (4) is defined by a smaller opening on a first side from which the light beam exits the window (4) and a larger opening on a second side from which the light beam enters the window (4);
the imaging element (2) is arranged such that the imaging surface (3) of the imaging element (2) is located over the first side and facing toward the first side of the window (4);
the apparatus further comprising a cover glass (6) fitted within the window (4) of the rigid circuit substrate (1) for covering the second side of the window (4); so that the window (4) of the rigid circuit substrate (1) spatially separates the cover glass (6) from the imaging surface (3) of the imaging element (2) and defines an air layer between the cover glass (6) and the imaging surface (3).

2. The imaging apparatus of claim 1, wherein the imaging surface (3) of the imaging element (2) has micro-lenses provided thereon, for causing a micro-lens effect due to a difference between an index of refraction of the micro-lenses and an index of refraction of the air layer defined between the cover glass (6) and the imaging surface (3).

3. The imaging apparatus of claim 1, wherein the rigid circuit substrate (1) has a central plate portion on which the window (4) is formed, and side bank portions projecting away from the first side of the central plate portion for defining a position for mounting the imaging element (2) on the central plate portion over the first side of the window (4).

4. The imaging apparatus of claim 1, wherein the cover glass (6) has a size sufficiently larger than that of the imaging surface (3) of the imaging element (2) such that end surface reflections of the light beam entering through the window (4) of the rigid circuit substrate (1) due to the cover glass (6) can be kept out of the imaging surface (3) of the imaging element (2).

5. The imaging apparatus of claim 1, wherein the rigid circuit substrate (1) has connection wirings embedded within the rigid circuit substrate (1), wherein the connection wirings are connected with the substrate connection pads (10).

6. The imaging apparatus of claim 1, wherein the rigid circuit substrate (1) is made of a ceramic material.

7. An imaging apparatus for an electronic endoscope, comprising:
a rigid circuit substrate (1) having electrodes (9) and a window (4) through which the light beam passes;
an imaging element (2) having an imaging surface (3), located on one side of the imaging element (2), for receiving the light beam through the window (4) of the rigid circuit substrate (1), the imaging element (2) being mounted on the rigid circuit substrate (1) by a face-down bonding;
CHARACTERIZED IN THAT
an insulative flexible print substrate (8) is connected with the electrodes (9) of the rigid circuit substrate (1);
the window (4) is defined by a smaller opening on a first side from which the light beam exits the window (4) and a larger opening on a second side from which the light beam enters the window (4);
the imaging surface (3) of the imaging element (2) is facing toward the first side and located over the first side of the window (4) of the rigid circuit substrate (1);
the apparatus further comprises a cover glass (6) fitted within the window (4) of the rigid circuit substrate (1) for covering the second side of the window (4); so that the window (4) of the rigid circuit substrate (1) spatially separates the cover glass (6) from the imaging surface (3) of the imaging element (2) and defines an air layer between the cover glass (6) and the imaging surface (3).

8. The imaging apparatus of claim 7, wherein the imaging surface (3) of the imaging element (2) has micro-lenses provided thereon, for causing a micro-lens effect due to a difference between an index of refraction of the micro-lenses and an index of refraction of the air layer defined between the cover glass (6) and the imaging surface (3).

9. The imaging apparatus of claim 7, wherein the rigid circuit substrate (1) has a central plate portion on which the window (4) is formed, and side bank portions projecting away from the first side of the central plate portion for defining a position for mounting the imaging element (2) on the central plate portion over the first side of the window (4).

10. The imaging apparatus of claim 7, wherein the cover glass (6) has a size sufficiently larger than that of the imaging surface (3) of the imaging element (2) such that end surface reflections of the light beam entering through the window (4) of the rigid circuit substrate (1) due to the cover glass (6) can be kept out of the imaging surface (3) of the imaging element (2).

11. The imaging apparatus of claim 7, wherein the rigid circuit substrate (1) has connection wirings embedded within the rigid circuit substrate (1), and substrate connection pads (10) connected with the connection wirings.

12. The imaging apparatus of claim 7, wherein the rigid circuit substrate (1) is made of a ceramic material.

## Patentansprüche

1. Abbildungsgerät für ein elektronisches Endoskop, mit:
einem steifen Schaltungssubstrat (1), das Substrat-Anschlußflächen (10), Elektroden (9), die mit den Anschlußflächen verbunden sind, und ein Fenster (4) aufweist, durch das ein Lichtstrahl hindurchtritt,
einem Abbildungselement (2), das eine Abbildungsfläche (3) für den Empfang des durch das Fenster (4) des steifen Schaltungssubstrats (1) hindurchtretenden Lichtstrahls sowie Element-Anschlußflächen (11) besitzt, wobei sowohl die Abbildungsfläche als auch die Element-Anschlußflächen auf einer Seite des Abbildungselements (2) angeordnet sind und das Abbildungselement (2) an dem steifen Schaltungssubstrat (1) derart angebracht ist, daß die Substrat-Anschlußflächen (10) des steifen Schaltungssubstrats (1) und die ElementAnschlußflächen (11) des Abbildungselements (2) elektrisch mittels einer gegenseitigen Druckverbindung zwischen dem steifen Schaltungssubstrat (1) und dem Abbildungselement (2) verbunden sind,
dadurch **gekennzeichnet**,
daß ein isolierendes flexibles Schaltungssubstrat (8) mit den Elektroden (9) des steifen Schaltungssubstrats (1) verbunden ist,
daß das Fenster (4) durch eine kleinere Öffnung auf einer ersten Seite, auf der der Lichtstrahl aus dem Fenster (4) austritt, und durch eine größere Öffnung auf einer zweiten Seite, auf der der Lichtstrahl in das Fenster (4) eintritt, definiert ist,
daß das Abbildungselement (2) derart angeordnet ist, daß die Abbildungsfläche (3) des Abbildungselements (2) über der ersten Seite angeordnet und zu der ersten Seite des Fensters (4) gerichtet ist,
daß das Gerät weiterhin ein Abdeckglas (6) aufweist, das in das Fenster (4) des steifen Schaltungssubstrats (1) eingepaßt ist und zur Abdeckung zur zweiten Seite des Fensters (4) dient, so daß das Fenster (4) des steifen Schaltungssubstrats (1) das Abdeckglas (6) räumlich von der Abbildungsfläche (3) des Abbildungselements (2) trennt und eine Luftschicht zwischen dem Abdeckglas (6) und der Abbildungsfläche (3) definiert.

2. Abbildungsgerät nach Anspruch 1, bei dem die Abbildungsfläche (3) des Abbildungselements (2) Mikrolinsen aufweist, die auf dieser vorgesehen sind und zur Erzielung eines Mikrolinsen-Effekts aufgrund eines Unterschieds zwischen einem Brechungsindex der Mikrolinsen und einem Brechungsindex der Luftschicht, die zwischen dem Abdeckglas (6) und der Abbildungsfläche (3) definiert ist, dienen.

3. Abbildungsgerät nach Anspruch 1, bei dem das steife Schaltungssubstrat (1) einen zentralen Plattenabschnitt besitzt, auf dem das Fenster (4) gebildet ist, und seitliche Bankabschnitte enthält, die von der ersten Seite des zentralen Plattenabschnitts wegweisend vorspringen und zur Definierung einer Position für die Montage des Abbildungselements (2) an dem zentralen Plattenabschnitt über der ersten Seite des Fensters (4) dienen.

4. Abbildungsgerät nach Anspruch 1, bei dem das Abdeckglas (6) eine Größe besitzt, die ausreichend größer ist als die der Abbildungsfläche (3) des Abbildungselements (2), so daß an den Endflächen auftretende Reflexionen des Lichtstrahls, der durch das Fenster (4) des steifen Schaltungssubstrats (1) eintritt, die durch das Abdeckglas (6) hervorgerufen werden, außerhalb der Abbildungsfläche (3) des Abbildungselements (2) gehalten werden können.

5. Abbildungsgerät nach Anspruch 1, bei dem das steife Schaltungssubstrat (1) Verbindungsverdrahtungen besitzt, die in das steife Schaltungssubstrat (1) eingebettet sind, wobei die Verbindungsverdrahtungen mit den Anschluliflächen (10) des Substrats verbunden sind.

6. Abbildungsgerät nach Anspruch 1, bei dem das steife Schaltungssubstrat (1) aus einem keramischen Material besteht.

7. Abbildungsgerät für ein elektronisches Endoskop, mit
einem steifen Schaltungssubstrat (1), das Elektroden (9) und ein Fenster (4), durch das der Lichtstrahl hindurchgeht, aufweist,
einem Abbildungselement (2) mit einer Abbildungsfläche (3), die an einer Seite des Abbildungselements (2) angeordnet ist und zum Aufnehmen des Lichstrahls durch das Fenster (4) des steifen Schaltungssubstrats (1) hindurch dient, wobei das Abbildungselement (2) an dem steifen Schaltungssubstrat (1) durch eine nach unten gewandte Bondverbindung angebracht ist,
dadurch **gekennzeichnet,**
daß ein isolierendes flexibles Schaltungssubstrat (8) mit den Elektroden (9) des steifen Schaltungssubstrats (1) verbunden ist,
daß das Fenster (4) durch eine kleinere Öffnung auf einer ersten Seite, auf der der Lichtstrahl aus dem Fenster (4) austritt, und durch eine größere Öffnung auf einer zweiten Seite, auf der der Lichtstrahl in das Fenster (4) eintritt, definiert ist,
daß die Abbildungsfläche (3) des Abbildungselement (2) der ersten Seite zugewandt ist und über der ersten Seite des Fensters (4) des steifen Schaltungssubstrats (1) angeordnet ist,
daß das Gerät weiterhin ein Abdeckglas (6) aufweist, das in das Fenster (4) des steifen Schaltungssubstrats (1) eingepaßt ist und zum Abdecken der zweiten Seite des Fensters (4) dient, so daß das Fenster (4) des steifen Schaltungssubstrats (1) das Abdeckglas (6) räumlich von der Abbildungsfläche (3) des Abbildungselements (2) trennt und eine Luftschicht zwischen dem Abdeckglas (6) und der Abbildungsfläche (3) definiert.

8. Abbildungsgerät nach Anspruch 7, bei dem die Abbildungsfläche (3) des Abbildungselements (2) Mikrolinsen besitzt, die auf dieser vorgesehen sind und dazu dienen, einen Mikrolinsen-Effekts aufgrund eines Unterschieds zwischen einem Brechungsindex der Mikrolinsen und einem Brechungsindex der Luftschicht, die zwischen dem Abdeckglas (6) und der Abbildungsfläche (3) vorhanden ist, hervorzurufen.

9. Abbildungsgerät nach Anspruch 7, bei dem das steife Schaltungssubstrat (1) einen zentralen Plattenabschnitt besitzt, auf dem das Fenster (4) gebildet ist, und seitliche Bankabschnitte enthält, die von der ersten Seite des zentralen Plattenabschnitts wegweisend vorstehen und zur Festlegung einer Position für die Anbringung des Abbildungselements (2) an dem zentralen Plattenabschnitt über der ersten Seite des Fensters (4) dienen.

10. Abbildungsgerät nach Anspruch 7, bei dem das Abdeckglas (6) eine Größe besitzt, die ausreichend größer ist als diejenige der Abbildungsfläche (3) des Abbildungselements (2), so daß durch Endflächen bedingte Reflexionen des Lichtstrahls, der durch das Fenster (4) des steifen Schaltungssubstrats (1) einfällt, die durch das Abdeckglas (6) hervorgerufen werden, außerhalb der Abbildungsfläche (3) des Abbildungselements (2) gehalten werden können.

11. Abbildungsgerät nach Anspruch 7, bei dem das steife Schaltungssubstrat (1) Verbindungsverdrahtungen aufweist, die in dem steifen Schaltungssubstrat (1) eingebettet sind, sowie Substrat-Anschlußflächen (10) enthält, die mit den Verbindungsverdrahtungen verbunden sind.

12. Abbildungsgerät nach Anspruch 7, bei dem das steife Schaltungssubstrat (1) aus einem keramischen Material besteht.

## Revendications

1. Appareil de prise de vue pour un endoscope électronique, comprenant :
un substrat rigide de circuit (1), ayant des plages de connexion de substrat (10), des électrodes (9) reliées aux plages de connexion, et une fenêtre (4) à travers laquelle un faisceau de lumière passe ;
un élément de prise de vue (2) ayant une surface de prise de vue (3) pour recevoir le faisceau de lumière à travers la fenêtre (4) du substrat rigide de circuit (1), et des plages de connexion d'élément (11), la surface de prise de vue et les plages de connexion d'élément étant situées les deux d'un côté de l'élément de prise de vue (2), l'élément de prise de vue (2) étant fixé sur le substrat rigide de circuit (1) de telle sorte que les plages de connexion de substrat (10) du substrat rigide de circuit (1) et les plages de connexion d'élément (11) de l'élément de prise de vue (2) sont reliés électriquement par une soudure par compression mutuelle entre le substrat rigide de circuit (1) et l'élément de prise de vue (2) ;
caractérisé en ce que
un substrat isolant imprimé souple (8) est relié aux électrodes (9) du substrat rigide de circuit (1) ;
la fenêtre (4) est définie par une ouverture plus petite sur un premier côté par laquelle le faisceau de lumière sort de la fenêtre (4) et une ouverture plus grande sur un second côté par laquelle le faisceau de lumière entre dans la fenêtre (4) ;
l'élément de prise de vue (2) est disposé de telle sorte que la surface de prise de vue (3) de l'élément de prise de vue (2) est situé sur le premier côté et en regard du premier côté de la fenêtre (4) ;
l'appareil comprenant en outre un verre de protection (6) rapporté dans la fenêtre (4) du substrat rigide de circuit (1) pour couvrir le second côté de la fenêtre (4) ; pour que la fenêtre (4) du substrat rigide de circuit (1) soit séparée spacialement par le verre de protection (6) de la surface de prise de vue (3) de l'élément de prise de vue (2) et définisse une couche d'air entre le verre de protection (6) et la surface de prise de vue (3).

2. Appareil de prise de vue selon la revendication 1, dans lequel la surface de prise de vue (3) de l'élément de prise de vue (2) a des micro-lentilles placées sur celle-ci, pour provoquer un effet de micro-lentille dû à une différence entre un indice de réfraction des micro-lentilles et un indice de réfraction de la couche d'air définie entre le verre de protection (6) et la surface de prise de vue (3).

3. Appareil de prise de vue selon la revendication 1, dans lequel le substrat rigide de circuit (1) a une partie plate centrale sur laquelle la fenêtre (4) est formée, et des parties latérales de groupe faisant saillie en s'éloignant du premier côté de la partie plate centrale pour définir une position pour monter l'élément de prise de vue (2) sur la partie plate centrale sur le premier côté de la fenêtre (4).

4. Appareil de prise de vue selon la revendication 1, dans lequel le verre de protection (6) a une taille suffisamment plus grande que celle de la surface de prise de vue (3) de l'élément de prise de vue (2) de telle sorte que des réflexions de surface d'extrémité du faisceau de lumière entrant à travers la fenêtre (4) du substrat rigide de circuit (1) dues au verre de protection (6) peuvent être maintenues en dehors de la surface de prise de vue (6) de l'élément de prise de vue (2).

5. Appareil de prise de vue selon la revendication 1, dans lequel le substrat rigide de circuit (1) a des conducteurs de connexion incorporés dans le substrat rigide de circuit (1), dans lequel les conducteurs de connexion sont reliés aux plages de connexion de substrat (10).

6. Appareil de prise de vue selon la revendication 1, dans lequel le substrat rigide de circuit (1) est fait d'un matériau en céramique.

7. Appareil de prise de vue pour un endoscope électronique, comprenant :
un substrat rigide de circuit (1), ayant des électrodes (9) et une fenêtre (4) à travers laquelle un faisceau de lumière passe ;
un élément de prise de vue (2) ayant une surface de prise de vue (3), situé d'un côté de l'élément de prise de vue (2), pour recevoir le faisceau de lumière à travers la fenêtre (4) du substrat rigide de circuit (1), l'élément de prise de vue (2) étant monté sur le substrat du circuit rigide (1) par une soudure avec la face en dessous ;
caractérisé en ce que
un substrat imprimé souple isolant (8) est relié aux électrodes (9) du substrat rigide de circuit (1) ;
la fenêtre (4) est définie par une ouverture plus petite sur un premier côté par laquelle le faisceau de lumière sort de la fenêtre (4) et une ouverture plus grande sur un second côté par laquelle le faisceau de lumière entre dans la fenêtre (4) ;
la surface de prise de vue (3) de l'élément de prise de vue (2) est en regard du premier côté et situé sur le premier côté de la fenêtre (4) du substrat rigide de circuit (1) ;
l'appareil comprend en outre un verre de protection (6) rapporté dans la fenêtre (4) du substrat rigide de circuit (1) pour couvrir le second côté de la fenêtre (4) ; pour que la fenêtre (4) du substrat rigide de circuit (1) soit séparée spacialement par le verre de protection (6) de la surface de prise de vue (3) de l'élément de prise de vue (2) et définisse une couche d'air entre le verre de protection (6) et la surface de prise de vue (3).

8. Appareil de prise de vue selon la revendication 7, dans lequel la surface de prise de vue (3) de l'élément de prise de vue (2) a des micro-lentilles placées sur celle-ci, pour provoquer un effet de micro-lentille dû à une différence entre un indice de réfraction des micro-lentilles et un indice de réfraction de la couche d'air définie entre le verre de protection (6) et la surface de prise de vue (3).

9. Appareil de prise de vue selon la revendication 7, dans lequel le substrat rigide de circuit (1) a une partie plate centrale sur laquelle la fenêtre (4) est formée, et des parties latérales de groupe faisant saillie en s'éloignant du premier côté de la partie plate centrale pour définir une position pour monter l'élément de prise de vue (2) sur la partie plate centrale sur le premier côté de la fenêtre (4).

10. Appareil de prise de vue selon la revendication 7, dans lequel le verre de protection (6) a une taille suffisamment plus grande que celle de la surface de prise de vue (3) de l'élément de prise de vue (2) de telle sorte que des réflexions de surface d'extrémité du faisceau de lumière entrant à travers la fenêtre (4) du substrat rigide de circuit (1) dues au verre de protection (6) peuvent être maintenues en dehors de la surface de prise de vue (6) de l'élément de prise de vue (2).

11. Appareil de prise de vue selon la revendication 7, dans lequel le substrat rigide de circuit (1) a des conducteurs de connexion incorporés dans le substrat rigide de circuit (1), et les plages de connexion de substrat (10) reliées aux conducteurs de connexion.

12. Appareil de prise de vue selon la revendication 7, dans lequel le substrat rigide de circuit (1) est fait d'un matériau en céramique.
